# EUROPÄISCHE PATENTSCHRIFT

(11) **EP 4 070 327 B1**
(45) Veröffentlichungstag und Bekanntmachung des Hinweises auf die Patenterteilung: **17.12.2025**
(21) Anmeldenummer: 20807798.2
(22) Anmeldetag: 23.11.2020
(51) Int. Cl.: G16H 30/40, G16H 50/20

(54) **UNTERSTÜTZUNG BEI DER ERKENNUNG VON LUNGENERKRANKUNGEN**
ASSISTANCE WITH DETECTION OF LUNG DISEASES
AIDE À LA DÉTECTION DES MALADIES PULMONAIRES

(30) Priorität: 05.12.2019 EP 19213939; 10.01.2020 EP 20151108
(43) Veröffentlichungstag der Anmeldung: 12.10.2022
(73) Patentinhaber: Bayer Aktiengesellschaft, 51373 Leverkusen (DE)
(72) Erfinder: GEHRE, Anne Claudia, 41515 Grevenbroich (DE); LABITZKE, Björn, 50931 Köln (DE); SHAFIN, Abdullah, 51381 Leverkusen (DE); DIEDAM, Holger, 50674 Köln (DE)
(74) Vertreter: BIP Patents
(86) Internationale Anmeldenummer: PCT/EP2020/083003
(87) Internationale Veröffentlichungsnummer: WO 2021/110446

(56) Entgegenhaltungen:
- WO-A1-2019/063520
- US-A1- 2011 029 248
- CHOI JUN-HO ET AL: "EmbraceNet: A robust deep learning architecture for multimodal classification", INFORMATION FUSION, vol. 51, 25 February 2019 (2019-02-25), pages 259 - 270, XP085685672, ISSN: 1566-2535, DOI: 10.1016/J.INFFUS.2019.02.010
- ANONYMOUS: "Introduction to Multimodal Learning Model - DEV Community 👩?💻👨?💻", 5 February 2019 (2019-02-05), XP055691056, Retrieved from the Internet <URL:https://dev.to/kayis/introduction-to-multimodal-learning-model-4ngm> [retrieved on 20200430]

## Beschreibung

Die vorliegende Erfindung betrifft die Erkennung des akuten Atemnotsyndroms bei einem Patienten. Gegenstände der vorliegenden Erfindung sind ein Computersystem, ein Verfahren und ein Computerprogrammprodukt zur Erkennung eines akuten Atemnotsyndroms.

Das akute Atemnotsyndrom (engl.: *Acute Respiratory Distress Syndrome,* kurz: ARDS) ist eine lebensgefährliche Erkrankung, bei der die Lunge nicht richtig arbeiten kann. ARDS wird durch Schäden in der Kapillarwand hervorgerufen, die auf eine Erkrankung oder physische Verletzung zurückgeführt werden können. Durch diese Schäden wird die Kapillarwand undicht, was zu einem Flüssigkeitsstau und letztlich zum Kollaps der Lungenbläschen führt. Dadurch ist die Lunge nicht länger in der Lage, Sauerstoff und Kohlendioxid auszutauschen. ARDS tritt normalerweise nicht als eigenständige Erkrankung auf, sondern ist die Folge einer anderen Erkrankung bzw. eines schwerwiegenden Unfalls oder einer Verletzung.

Obwohl jeder zehnte Intensivpatient an einem akuten Lungenversagen leidet, wird die lebensgefährliche Komplikation häufig nicht erkannt (JAMA 2016; 315: 788-800). Der drohende Tod kann häufig durch eine Reihe von einfachen Interventionen verhindert werden; zu ihnen gehört die frühzeitige mechanische Beatmung mit positiv-endexspiratorischem Druck (PEEP) und einem reduzierten Tidalvolumen. Empfohlen wird zudem eine Bauchlage des Patienten. Diese Maßnahmen haben in klinischen Studien die Mortalität teilweise deutlich gesenkt. Diese Empfehlungen können jedoch nur umgesetzt werden, wenn ARDS frühzeitig erkannt wird.

Es gibt zahlreiche Publikationen zur automatisierten Erkennung von Lungenerkrankungen auf Basis von Patientendaten. US2011029248A1 offenbart eine Vorrichtung zur Vorhersage der Atemstabilität eines Patienten, mit einem Patientendaten-Speicher, der Patientendaten für einen Patienten speichert, und einem Analysegerät, das mit dem Speicher in Verbindung steht und einen Maßwert für die Atemstabilität des Patienten berechnet. P. Rajpurkar *et al.* beschreiben ein künstliches neuronales Netz zur Erkennung einer Lungenentzündung anhand von Röntgenaufnahmen des Brustbereichs von Patienten (CheXNet: *Radiologist-Level Pneumonia Detection on Chest X-Rays with Deep Learning;* arXiv:1711.05225 [cs.CV]). ARDS lässt sich jedoch zuverlässig nicht allein anhand von Röntgenaufnahmen erkennen. WO2013/121374A2 offenbart ein System zum Erkennen von ARDS bei einem Patienten, das physiologische Parameter des Patienten analysiert. Physiologische Parameter alleine erlauben jedoch keine sichere Erkennung von ARDS.

Ausgehend vom beschriebenen Stand der Technik bestand die technische Aufgabe in der Bereitstellung einer Lösung zur sicheren Erkennung von ARDS bei Intensivpatienten.

Diese Aufgabe wird von den Gegenständen der unabhängigen Patentansprüche gelöst. Bevorzugte Ausführungsformen finden sich in den abhängigen Patentansprüchen sowie in der vorliegenden Beschreibung und in den Zeichnungen.

Ein erster Gegenstand der vorliegenden Erfindung ist ein Computersystem umfassend
- eine Eingabeeinheit,
- eine Steuer- und Recheneinheit und
- eine Ausgabeeinheit

wobei die Steuer- und Recheneinheit konfiguriert ist, die Eingabeeinheit zu veranlassen, Patientendaten zu einem Intensivpatienten zu empfangen, wobei die Patientendaten mindestens die folgenden Patientendaten umfassen:
   ∘ eine Mehrzahl an radiologischen Aufnahmen vom Thorax des Intensivpatienten, wobei die radiologischen Aufnahmen den Thorax zu unterschiedlichen Zeitpunkten zeigen, und
   ∘ eine Mehrzahl an Vitaldaten zu Vitalparametern des Intensivpatienten, wobei die Vitaldaten Werte zu den Vitalparametern zu unterschiedlichen Zeitpunkten angeben,
wobei die Steuer- und Recheneinheit konfiguriert ist, die empfangenen Patientendaten einem künstlichen neuronalen Netz zuzuführen,
   ∘ wobei das künstliche neuronale Netz mindestens drei Teilnetze umfasst, ein erstes Teilnetz, ein zweites Teilnetz und ein drittes Teilnetz,
   ∘ wobei das erste Teilnetz eine erste Eingabeschicht umfasst, wobei das zweite Teilnetz eine zweite Eingabeschicht umfasst, wobei das dritte Teilnetz eine Ausgabeschicht umfasst, und wobei das erste Teilnetz und das zweite Teilnetz in dem dritten Teilnetz zusammengeführt werden,
   ∘ wobei die Mehrzahl an radiologischen Aufnahmen der ersten Eingabeschicht zugeführt wird und die Mehrzahl an Vitaldaten der zweiten Eingabeschicht zugeführt wird,
   ∘ wobei das erste Teilnetz konfiguriert ist, für jede radiologische Aufnahme einen zeitabhängigen Bild-Deskriptor zu erzeugen,
   ∘ wobei das zweite Teilnetz konfiguriert ist, aus den Vitaldaten zeitabhängige Vitaldaten-Deskriptoren zu erzeugen,
   ∘ wobei die zeitabhängigen Bild-Deskriptoren und die zeitabhängigen Vitaldaten-Deskriptoren Schichten in dem künstlichen neuronalen Netz zugeführt werden, die rückgekoppelte Neuronen umfassen,
   ∘ wobei das künstliche neuronale Netz mittels Referenzdaten trainiert worden ist, anhand von Patientendaten einen ARDS-Indikator-Wert zu berechnen und den ARDS-Indikator-Wert über die Ausgangsschicht auszugeben,
wobei die Steuer- und Recheneinheit konfiguriert ist, den ARDS-Indikator-Wert von dem künstlichen neuronalen Netz zu empfangen,
wobei die Steuer- und Recheneinheit konfiguriert ist, den ARDS-Indikator-Wert mit einem Schwellenwert zu vergleichen, und
wobei die Steuer- und Recheneinheit konfiguriert ist, die Ausgabeeinheit zu veranlassen, eine Mitteilung auszugeben, wenn der ARDS-Indikator-Wert in definierter Weise von dem Schwellenwert abweicht.

Ein weiterer Gegenstand der vorliegenden Erfindung ist ein Verfahren zur Erkennung von ARDS bei einem Intensivpatienten umfassend die Schritte
- Empfangen von Patientendaten zu dem Intensivpatienten, wobei die Patientendaten mindestens die folgenden Patientendaten umfassen:
   ∘ eine Mehrzahl an radiologischen Aufnahmen vom Thorax des Intensivpatienten, wobei die radiologischen Aufnahmen den Thorax zu unterschiedlichen Zeitpunkten zeigen, und
   ∘ eine Mehrzahl an Vitaldaten zu Vitalparametern des Intensivpatienten, wobei die Vitaldaten Werte zu den Vitalparametern zu unterschiedlichen Zeitpunkten angeben,
- Zuführen der Patientendaten einem künstlichen neuronalen Netz,
   ∘ wobei das künstliche neuronale Netz mindestens drei Teilnetze umfasst, ein erstes Teilnetz, ein zweites Teilnetz und ein drittes Teilnetz,
   ∘ wobei das erste Teilnetz eine erste Eingabeschicht umfasst, wobei das zweite Teilnetz eine zweite Eingabeschicht umfasst, wobei das dritte Teilnetz eine Ausgabeschicht umfasst, und wobei das erste Teilnetz und das zweite Teilnetz in dem dritten Teilnetz zusammengeführt werden,
   ∘ wobei die Mehrzahl an radiologischen Aufnahmen der ersten Eingabeschicht zugeführt wird und die Mehrzahl an Vitaldaten der zweiten Eingabeschicht zugeführt wird,
   ∘ wobei das erste Teilnetz konfiguriert ist, für jede radiologische Aufnahme einen zeitabhängigen Bild-Deskriptor zu erzeugen,
   ∘ wobei das zweite Teilnetz konfiguriert ist, aus den Vitaldaten zeitabhängige Vitaldaten-Deskriptoren zu erzeugen,
   ∘ wobei die zeitabhängigen Bild-Deskriptoren und die zeitabhängigen Vitaldaten-Deskriptoren Schichten in dem künstlichen neuronalen Netz zugeführt werden, die rückgekoppelte Neuronen umfassen,
   ∘ wobei das künstliche neuronale Netz mittels Referenzdaten trainiert worden ist, anhand von Patientendaten einen ARDS-Indikator-Wert zu berechnen und den ARDS-Indikator-Wert über die Ausgangsschicht auszugeben,
- Empfangen eines ARDS-Indikator-Wertes für die zugeführten Patientendaten von dem künstlichen neuronalen Netz,
- Vergleichen des ARDS-Indikator-Werts mit einem Schwellenwert,
- Ausgeben einer Mitteilung für den Fall, dass der ARDS-Indikator-Wert in definierter Weise von dem Schwellenwert abweicht.

Ein weiterer Gegenstand der vorliegenden Erfindung ist ein Computerprogrammprodukt umfassend ein Computerprogramm, das in einen Arbeitsspeicher eines Computersystems geladen werden kann und dort das Computersystem dazu veranlasst, folgende Schritte ausführen:
- Empfangen von Patientendaten zu dem Intensivpatienten, wobei die Patientendaten mindestens die folgenden Patientendaten umfassen:
   ∘ eine Mehrzahl an radiologischen Aufnahmen vom Thorax des Intensivpatienten, wobei die radiologischen Aufnahmen den Thorax zu unterschiedlichen Zeitpunkten zeigen, und
   ∘ eine Mehrzahl an Vitaldaten zu Vitalparametern des Intensivpatienten, wobei die Vitaldaten Werte zu den Vitalparametern zu unterschiedlichen Zeitpunkten angeben,
- Zuführen der Patientendaten einem künstlichen neuronalen Netz,
   ∘ wobei das künstliche neuronale Netz mindestens drei Teilnetze umfasst, ein erstes Teilnetz, ein zweites Teilnetz und ein drittes Teilnetz,
   ∘ wobei das erste Teilnetz eine erste Eingabeschicht umfasst, wobei das zweite Teilnetz eine zweite Eingabeschicht umfasst, wobei das dritte Teilnetz eine Ausgabeschicht umfasst, und wobei das erste Teilnetz und das zweite Teilnetz in dem dritten Teilnetz zusammengeführt werden,
   ∘ wobei die Mehrzahl an radiologischen Aufnahmen der ersten Eingabeschicht zugeführt wird und die Mehrzahl an Vitaldaten der zweiten Eingabeschicht zugeführt wird,
   ∘ wobei das erste Teilnetz konfiguriert ist, für jede radiologische Aufnahme einen zeitabhängigen Bild-Deskriptor zu erzeugen,
   ∘ wobei das zweite Teilnetz konfiguriert ist, aus den Vitaldaten zeitabhängige Vitaldaten-Deskriptoren zu erzeugen,
   ∘ wobei die zeitabhängigen Bild-Deskriptoren und die zeitabhängigen Vitaldaten-Deskriptoren Schichten in dem künstlichen neuronalen Netz zugeführt werden, die rückgekoppelte Neuronen umfassen,
   ∘ wobei das künstliche neuronale Netz mittels Referenzdaten trainiert worden ist, anhand von Patientendaten einen ARDS-Indikator-Wert zu berechnen und den ARDS-Indikator-Wert über die Ausgangsschicht auszugeben,
- Empfangen eines ARDS-Indikator-Wertes für die zugeführten Patientendaten von dem künstlichen neuronalen Netz,
- Vergleichen des ARDS-Indikator-Werts mit einem definierten Schwellenwert,
- Ausgeben einer Mitteilung für den Fall, dass der ARDS-Indikator-Wert in definierter Weise von dem Schwellenwert abweicht.

Die Erfindung wird nachstehend näher erläutert, ohne zwischen den Erfindungsgegenständen (Computersystem, Verfahren, Computerprogrammprodukt) zu unterscheiden. Die nachfolgenden Erläuterungen sollen vielmehr für alle Erfindungsgegenstände in analoger Weise gelten, unabhängig davon, in welchem Kontext (Computersystem, Verfahren, Computerprogrammprodukt) sie erfolgen.

Sind in der vorliegenden Beschreibung Schritte in einer bestimmten Reihenfolge aufgeführt, so bedeutet dies nicht zwangsläufig, dass die Schritte auch in der angegebenen Reihenfolge ausgeführt werden müssen. Die Erfindung soll vielmehr so verstanden werden, dass die in einer bestimmten Reihenfolge aufgeführten Schritte in einer beliebigen Reihenfolge oder auch parallel zueinander ausgeführt werden können, es sei denn, ein Schritt basiert auf einem anderen Schritt, was aus der Beschreibung der Schritte jeweils deutlich wird. Die konkret in diesem Dokument aufgeführten Reihenfolgen stellen demnach nur bevorzugte Ausführungsformen der Erfindung dar.

Die vorliegende Erfindung stellt einem Arzt und/oder dem Krankenhauspersonal Mittel bereit, die den Arzt und/oder das Krankenhauspersonal dabei unterstützen, das Eintreten eines akuten Atemnotsyndroms (ARDS) beim einem Intensivpatienten zu erkennen.

Dabei wird/werden der Arzt und/oder das Krankenhauspersonal durch ein Computersystem unterstützt.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher und dergleichen. Auch interne Anschlüsse und Erweiterungskarten gelten in der Computertechnik als Peripherie.

Computersysteme von heute werden häufig in Desktop-PCs, Portable PCs, Laptops, Notebooks, Netbooks, Tablet-PCs, Handhelds (z.B. Smartphone), Cloud-Rechner und Workstations unterteilt; alle diese Systeme können prinzipiell zur Ausführung der Erfindung genutzt werden. Vorzugsweise wird die vorliegende Erfindung auf einem oder mehreren Computersystemen auf Intensivstationen von Krankhäusern ausgeführt. Es ist denkbar, dass ein solches Computersystem auf einer Intensivstation weitere Funktionen ausführt, wie beispielsweise die Überwachung des Gesundheitszustands von Intensivpatienten. Vorzugsweise kann das Computersystem auf Datenbanken des Krankenhauses zugreifen, in denen Patientendaten der Intensivpatienten gespeichert sein können. Vorzugsweise wird die Erfindung als Hintergrundprozess automatisiert auf einem oder mehreren Computersystemen ausgeführt.

Das erfindungsgemäße Computersystem ist konfiguriert, anhand von Patientendaten einen ARDS-Indikator-Wert zu ermitteln. Der ARDS-Indikator-Wert korreliert mit der Wahrscheinlichkeit, dass bei dem Intensivpatienten ein akutes Atemnotsyndrom vorliegt. Vorzugsweise gibt der ARDS-Indikator-Wert die Wahrscheinlichkeit an, dass bei dem Intensivpatienten ARDS vorliegt, wobei ein Wert von 0 bedeuten kann, dass ARDS ausgeschlossen werden kann, und ein Wert von 1 oder 100 % bedeuten kann, dass alle ausgewerteten Patientendaten darauf hindeuten und/oder den Schluss zulassen, dass ARDS vorliegt.

Das erfindungsgemäße Computersystem ist konfiguriert, den ARDS-Indikator-Wert mit einem definierten Schwellenwert zu vergleichen. Liegt eine definierte Abweichung zwischen dem ARDS-Indikator-Wert und dem Schwellenwert vor, gibt das Computersystem eine Mitteilung aus. Vorzugsweise ist das erfindungsgemäße Computersystem konfiguriert, dann eine Mitteilung auszugeben, wenn der ARDS-Indikator-Wert oberhalb des definierten Schwellenwerts liegt und damit die Wahrscheinlichkeit, dass ARDS bei dem Intensivpatienten vorliegt, den Schwellenwert überschritten hat. In einem solchen Fall sollten Maßnahmen getroffen werden, die eine Verschlechterung des Zustands des Patienten verhindern. Liegt der ARDS-Indikator-Wert unterhalb des definierten Schwellenwerts, so ist die Wahrscheinlichkeit, dass ARDS bei dem Intensivpatienten vorliegt, so gering, dass keine ARDS-spezifischen Maßnahmen erforderlich sind.

Der Schwellenwert kann beispielsweise ein Wert zwischen 0,5 (bzw. 50 %) und 1 sein. Vorzugsweise wird der Schwellenwert durch einen Arzt und/oder anhand medizinischer Erfahrungen, insbesondere auf Basis von Erfahrungen, die mit der vorliegenden Erfindung gewonnen werden können, festgelegt. So kann der Schwellenwert beispielsweise anfänglich bewusst niedrig angesetzt werden, um jegliches Risiko auszuschließen, das akute Atemnotsyndrom bei einem Patienten zu übersehen. Der Schwellenwert kann dann im Laufe der Zeit auf einen höheren Schwellenwert gesetzt werden, der aus medizinischer Sicht und Erfahrung angemessen erscheint.

Bei der Mitteilung kann es sich um eine Mitteilung auf einem Bildschirm (z.B. eine Textnachricht) und/oder ein akustisches Signal und/oder ein optisches Signal und/oder einen Vibrationsalarm und/oder dergleichen handeln. Die Mitteilung kann auf einem oder mehreren Ausgabeeinheiten des erfindungsgemäßen Computersystems ausgegeben werden, zum Beispiel auf einem Monitor angezeigt werden und/oder über einen Lautsprecher ausgegeben werden und/oder auf einem Drucker z.B. als Textnachricht ausgeben und/oder zum Beispiel über E-Mail an ein stationäres Computersystem oder ein mobiles Empfangsgerät (z.B. ein Smartphone oder ein Tabletcomputer oder an einen Funkmeldeempfänger (Pager)) übermittelt werden.

Die Mitteilung soll einen Arzt und/oder das Krankenhauspersonal veranlassen, sich dem Intensivpatienten zu widmen und Maßnahmen zu ergreifen, um eine Verschlechterung seines Gesundheitszustands zu verhindern. Vorzugsweise umfasst die Mitteilung Handlungsempfehlungen, die ein Arzt oder das Krankenhauspersonal ergreifen sollen, um eine Verschlechterung des Gesundheitszustands des Intensivpatienten zu verhindern.

Das erfindungsgemäße Computersystem ist konfiguriert, den ARDS-Indikator-Wert automatisiert zu ermitteln, den ARDS-Indikator-Wert automatisiert mit dem definierten Schwellenwert zu vergleichen und automatisiert eine oder mehrere Mitteilungen zu versenden.

"Automatisiert" bedeutet, dass keinerlei menschliches Zutun erforderlich ist.

Die Ermittlung des ARDS-Indikator-Werts erfolgt auf Basis von Patientendaten. Das erfindungsgemäße Computersystem kann Zugriff auf die Patientendaten haben und/oder dem Computersystem können die Patientendaten automatisiert zugeführt werden.

Die Patientendaten können zum Beispiel in einem oder mehreren Datenspeichern vorliegen, die Bestandteil des erfindungsgemäßen Computersystems sind oder mit diesem über ein Netzwerk oder über mehrere Netzwerke verbunden sind. Insbesondere kann es sich bei solchen Datenspeichern um eine oder mehrere Datenbanken eines Krankenhauses handeln, vorzugsweise um Datenbanken, in denen Patientendaten gespeichert sind. Das erfindungsgemäße Computersystem kann konfiguriert sein, zu definierten Zeitpunkten und/oder in definierten Zeitabständen und/oder bei Eintreten definierter Ereignisse (z.B. bei Vorliegen neuer Patientendaten) Patientendaten aus dem einen oder den mehreren Datenspeichern auszulesen und diese zur Ermittlung des ARDS-Indikator-Wertes zu verwenden.

Ebenso ist es denkbar, dass das erfindungsgemäße Computersystem konfiguriert ist, patientenspezifische Daten von medizinischen Geräten und/oder Computersystemen, die mit medizinischen Geräten verbunden sind, abzufragen / zu empfangen.

Unter dem Begriff "medizinische Geräte" werden Geräte verstanden, mit deren Hilfe physiologische Informationen über einen Patienten gewonnen werden können. Beispiele für solche medizinischen Geräte sind Herzfrequenzmesser, Blutdruckmesser, Fieberthermometer, Röntgenapparate, Computertomografen und dergleichen. Bei den medizinischen Geräten handelt es sich insbesondere um Röntgenapparate, Computertomografen, Beatmungsgeräte und Geräte, mit denen der arterielle Sauerstoffpartialdruck-Wert PaO₂ eines Patienten, der inspiratorische Sauerstoffkonzentrations-Wert FiO₂ und/oder das PaO₂/FiO₂-Verhältnis sowie PEEP- und CPAP-Werte (PEEP = Positive EndExpiratory Pressure; CPAP = Continuous Positive Airway Pressure) ermittelt werden können.

Vorzugsweise ist das erfindungsgemäße Computersystem konfiguriert, immer dann einen ARDS-Indikator-Wert zu ermitteln, wenn neue Patientendaten erfasst und in einem Datenspeicher, der Bestandteil des erfindungsgemäße Computersystems sein kann oder mit dem das erfindungsgemäße Computersystem verbunden sein kann, gespeichert werden. Das erfindungsgemäße Computersystem kann konfiguriert sein, in definierten Zeitabständen (z.B. alle 10 Minuten) zu prüfen, ob neue Patientendaten in dem Datenspeicher vorliegen.

Die Patientendaten, anhand der die Ermittlung des ARDS-Indikator-Werts erfolgt, umfassen eine Mehrzahl an radiologischen Aufnahmen des Thorax des Intensivpatienten.

Beispiele für radiologische Aufnahmen sind Röntgenbilder, CT-Scans (CT = Computertomografie) und dergleichen. Vorzugsweise werden Röntgenbilder verwendet. Bei ARDS-Patienten sind in radiologischen Aufnahmen beispielsweise bilaterale Verdichtungen in der Lunge zu erkennen (siehe z.B. H.M. Kulke: Röntgendiagnostik von Thoraxerkrankungen, De Gruyter Verlag 2013, ISBN 978-3-11-031118-1).

Die einzelnen radiologischen Aufnahmen zeigen den Thorax des Intensivpatienten zu unterschiedlichen Zeitpunkten. Vorzugsweise sind mindestens drei Aufnahmen vorhanden, die innerhalb der letzten sieben Tage erzeugt worden sind. Besonders bevorzugt wurde die jüngste radiologische Aufnahme innerhalb der letzten 24 Stunden, noch mehr bevorzugt innerhalb der letzten 12 Stunden, am meisten bevorzugt innerhalb der letzten drei Stunden erzeugt.

Vorzugsweise tragen die radiologischen Aufnahmen jeweils einen Zeitstempel, der anzeigt, wann sie erzeugt (messtechnisch erfasst) worden sind.

Die radiologischen Aufnahmen liegen vorzugsweise als digitale Bilddateien vor. Der Begriff "digital" bedeutet, dass die radiologischen Aufnahmen von einer Maschine, in der Regel einem Computersystem, verarbeitet werden können. Unter "Verarbeitung" werden die bekannten Verfahren zur elektronischen Datenverarbeitung (EDV) verstanden.

Digitale Bilddateien können in verschiedenen Formaten vorliegen. Digitale Bilddateien können beispielsweise als Rastergrafiken kodiert werden. Rastergrafiken bestehen aus einer rasterförmigen Anordnung von so genannten Bildpunkten (Pixel) oder Volumenelementen (Voxel), denen jeweils eine Farbe bzw. ein Grauwert zugeordnet ist. Zur Vereinfachung wird in dieser Beschreibung angenommen, dass die radiologischen Aufnahmen als Rastergrafiken vorliegen. Diese Annahme soll jedoch in keiner Weise einschränkend verstanden werden. Es gibt eine Vielzahl an möglichen digitalen Bildformaten und Farbkodierungen; dem Fachmann der Bildverarbeitung ist klar, wie er die Lehre dieser Beschreibung auf verschiedene Bildformate übertragen kann.

Weitere Patientendaten, die zur Ermittlung eines ARDS-Indikator-Wertes herangezogen werden, umfassen Vitaldaten zu Vitalparametern des Intensivpatienten.

"Vitalparameter" sind Messgrößen wichtiger Körperfunktionen, die bei der Kontrolle der Vitalzeichen festgestellt werden. Üblicherweise umfassen die Vitalparameter die Herzfrequenz, die Atemfrequenz, den Blutdruck und die Körpertemperatur des Intensivpatienten.

Weitere Vitalparameter sind die Sauerstoffsättigung im Blut und/oder der Sauerstoffpartialdruck.

Die Sauerstoffsättigung, kurz sO₂, ist der Quotient des im Blut vorhandenen Sauerstoffs und der maximalen Sauerstoffkapazität des Blutes in Prozent. Die Sauerstoffsättigung gibt also an, wieviel Prozent des gesamten Hämoglobins im Blut mit Sauerstoff beladen ist. Die Sauerstoffsättigung kann in verschiedenen Abschnitten des Herz-Kreislauf-Systems mit unterschiedlichen Verfahren bestimmt werden. Man kann folgende Sauerstoffsättigungen unterscheiden: arterielle Sauerstoffsättigung (SₐO₂), venöse Sauerstoffsättigung (SᵥO₂), zentralvenöse Sauerstoffsättigung (S_{zv}O₂) und gemischt-venöse Sauerstoffsättigung (S_{gv}O₂). Vorzugsweise wird die arterielle Sauerstoffsättigung und/oder die gemischt-venöse Sauerstoffsättigung erfasst. Die Sauerstoffsättigung wird vorzugsweise mit einem Pulsoxymeter und/oder durch eine Blutgasanalyse erfasst.

Als Sauerstoffpartialdruck (pO₂) wird der Druck bezeichnet, den der gasförmige Sauerstoff im Blut ausübt. Die Sauerstoffsättigung ist umso höher, je höher der Sauerstoffpartialdruck im Blut ist. Durch die Abhängigkeit der Sauerstoffaffinität des Hämoglobins von der Anzahl der bereits gebundenen O₂-Moleküle ist dieser Zusammenhang nicht-linear; die Sauerstoffbindungskurve zeigt einen s-förmigen Verlauf. Vorzugsweise wird der arterielle Sauerstoffpartialdruck (pₐO₂) erfasst.

Ein weiterer Vitalparameter ist die inspiratorische Sauerstofffraktion (FiO₂). Die inspiratorische Sauerstofffraktion gibt den Anteil des Sauerstoffs im Inspirationsgas an. Sie kann in Prozent oder als Dezimalzahl angegeben werden.

Ein weiterer Vitalparameter ist der Oxygenierungsindex (Horovitz-Quotient). Er ist definiert als Quotient aus dem arteriellen Sauerstoffpartialdruck (pₐO₂) und der Konzentration von Sauerstoff in der eingeatmeten Luft (FiO₂): Horovitz-Quotient = pₐO₂ / FiO₂.

Weitere Vitalparameter sind denkbar, insbesondere Vitalparameter, die sich aus den genannten Vitalparametern errechnen oder ableiten lassen.

Da bei Intensivpatienten regelmäßig eine Blutgasanalyse durchgeführt wird, können weitere Werte von Parametern, die bei der Blutgasanalyse ermittelt werden, als Vitalparameter in die automatisierte Erkennung von ARDS einfließen, wie beispielsweise der pH-Wert des Blutes, der Kohlenstoffdioxidpartialdruck, tatsächliches Bicarbonat, Basenüberschuss und/oder dergleichen (siehe z.B. H. W. Striebel: Anästhesie, Intensivmedizin, Notfallmedizin, 7. Aufl. Schattauer 2009, ISBN: 978-3-7945-2635-2). Analoges gilt für weitere Parameter, deren Werte routinemäßig bei Intensivpatienten ermittelt werden.

Die Vitaldaten geben die Werte der Vitalparameter zu unterschiedlichen Zeitpunkten wieder. Vorzugsweise sind für jeden Vitalparameter Werte zu mindestens zehn Zeitpunkten vorhanden, die vorzugsweise innerhalb der letzten 12 Stunden erzeugt worden sind. In einer Ausführungsform tragen die Vitaldaten jeweils einen Zeitstempel, der anzeigt, wann sie erfasst worden sind. In einer anderen Ausführungsform handelt es sich bei den Vitaldaten oder einem Teil der Vitaldaten um zeitliche Verlaufsdaten, d.h. Werte von Vitalparametern als Funktion der Zeit.

Es ist denkbar, dass weitere Daten der Ermittlung des ARDS-Indikator-Wertes zugrunde gelegt werden. Bei diesen weiteren Daten kann es sich um dynamische Daten handeln. Unter dem Begriff "dynamische Daten" werden Daten verstanden, die sich im Verlauf einer Zeitspanne (z.B. innerhalb einer Woche oder innerhalb eines Tages) signifikant ändern können. Die beschriebenen Vitaldaten zählen zu den dynamischen Daten. Bei den weiteren Daten kann es sich ferner um quasi-statische und/oder statische Daten handeln. Darunter werden Daten verstanden, die sich im Verlauf der Zeitspanne nicht signifikant (quasi-statisch) oder überhaupt nicht (statisch) ändern. Eine "signifikante Änderung" liegt dann vor, wenn die Änderung eine Bedeutung für den Krankheitsverlauf, insbesondere das mögliche Eintreten von ARDS hat. Ein Beispiel für ein statisches Datum ist das Geschlecht des Intensivpatienten. Ein Beispiel für ein quasi-statisches Datum ist das Alter oder das Gewicht oder die Größe des Intensivpatienten. Bei den weiteren Daten kann es sich auch um historische Daten handeln, wie beispielsweise das Ergebnis einer Diagnose, Anamnesedaten, Daten zur Selbsteinschätzung des Patienten und dergleichen.

Die Ermittlung des ARDS-Indikator-Wertes erfolgt mit Hilfe eines künstlichen neuronalen Netzes. Das künstliche neuronale Netz ist anhand von Referenzdaten trainiert worden, einen ARDS-Indikator-Wert auf Basis von Patientendaten zu ermitteln. Das Trainieren und das Validieren des künstlichen neuronalen Netzes können beispielsweise durch überwachtes Lernen erfolgt sein. Dabei wurden dem künstlichen neuronalen Netz Patientendaten präsentiert und es wurde dem künstlichen neuronalen Netz vermittelt, ob die Patienten, von denen die Patientendaten stammen, an ARDS leiden oder nicht an ARDS leiden. Anhand dieser Trainingsdaten kann zum Beispiel mittels eines Backpropagation-Verfahrens ein Modell erzeugt werden, das eine Beziehung zwischen den Patientendaten und der Diagnose (ARDS liegt vor oder liegt nicht vor) lernt, die auf unbekannte Patientendaten (Daten von Patienten, von denen man nicht genau weiß, ob sie an ARDS leiden) angewendet werden kann.

Das erfindungsgemäße künstliche neuronale Netz umfasst separate Eingabeschichten für die radiologischen Aufnahmen und für die Vitaldaten. Es sind demnach mindestens zwei Eingabeschichten vorhanden, eine erste Eingabeschicht für die Eingabe der radiologischen Aufnahmen und eine zweite Eingabeschicht für die Vitaldaten. Es ist auch denkbar, dass mehr als zwei Eingabeschichten vorhanden sind. Beispielsweise ist denkbar, dass für jede radiologische Aufnahme der vorliegenden Mehrzahl an radiologischen Aufnahmen eine separate Eingabeschicht vorhanden ist. Ferner ist denkbar, dass für jeden erfassten Vitalparameter eine separate Eingabeschicht für die entsprechenden Vitaldaten vorhanden ist. Ferner ist denkbar, dass weitere Eingabeschichten für weitere Daten vorhanden sind, beispielsweise für weitere dynamische, quasistatische und/oder statische Daten.

Zumindest die radiologischen Aufnahmen und die Vitaldaten werden von dem erfindungsgemäßen künstlichen neuronalen Netz zunächst separat voneinander verarbeitet. Ziel dieser separaten Verarbeitung ist die Ermittlung von separaten zeitabhängigen Deskriptoren. Das erfindungsgemäße künstliche neuronale Netz umfasst daher mindestens drei Teilnetze, ein erstes Teilnetz, ein zweites Teilnetz und ein drittes Teilnetz. Das erste Teilnetz dient der Ermittlung von zeitabhängigen Deskriptoren für die radiologischen Aufnahmen. Diese Deskriptoren werden in dieser Beschreibung auch als Bild-Deskriptoren bezeichnet. Das zweite Teilnetz dient der Ermittlung von zeitabhängigen Deskriptoren für die Vitaldaten. Diese Deskriptoren werden in dieser Beschreibung auch als Vitaldaten-Deskriptoren bezeichnet. Das erste Teilnetz und das zweite Teilnetz werden in dem dritten Teilnetz zusammengeführt. Unter Zusammenführung wird verstanden, dass mindestens eine Schicht des ersten Teilnetzes und mindestens eine Schicht des zweiten Teilnetzes jeweils mit einer Schicht des dritten Teilnetzes verbunden sind. Das dritte Teilnetz umfasst eine Ausgabeschicht. Die Ausgabeschicht dient der Ausgabe des ARDS-Indikator-Wertes.

Bei den Bild-Deskriptoren und den Vitaldaten-Deskriptoren handelt es sich jeweils um Repräsentationen der jeweiligen Daten. Ein Bild-Deskriptor ist eine Repräsentation einer radiologischen Aufnahme; ein Vitaldaten-Deskriptor ist eine Repräsentation von Werten eines Vitalparameters oder mehrerer Vitalparameter. Dabei verfügt der Deskriptor vorzugsweise über weniger Dimensionen als die ursprünglichen Daten. Bei der Erzeugung eines Deskriptors wird also eine Repräsentation der jeweiligen Daten erzeugt, die mit weniger Dimensionen auskommt, als die ursprünglichen Daten. Diese Dimensionsreduktion kann beispielsweise durch ein Convolutional Neural Network (CNN) erreicht werden. Das erste und/oder das zweite Teilnetz können demnach jeweils als CNN aufgebaut sein oder entsprechende Schichten (insbesondere Convolutional Layer und Pooling Layer) umfassen.

Das erste Teilnetz zur Erzeugung der Bild-Deskriptoren ist vorzugsweise ein Dense Convolutional Neural Network (DenseNet). Derartige Netze sind beispielsweise beschrieben in: G. Hunag et al.: Densely Connected Convolutional Networks, arXiv:1608.06993v5 [cs.CV] 28 Jan 2018. Auch das bereits oben erwähnte ChestXNet oder Teile davon kann/können als erstes Teilnetz verwendet werden.

Bei den Bild-Deskriptoren und den Vitaldaten-Deskriptoren handelt es sich um zeitabhängige Deskriptoren. Das bedeutet, dass die Informationen zu den Zeitpunkten, an denen die Daten, die dem Deskriptor zugrunde liegen, erfasst worden sind, zumindest teilweise noch in dem Deskriptor vorhanden sind oder diesem bei seiner weiteren Verarbeitung durch das erfindungsgemäße künstliche neuronale Netz beigegeben werden. Diese Zeitinformationen sind für die Ermittlung des ARDS-Indikator-Wertes wichtig und werden durch rückgekoppelte Neuronen verarbeitet. Das erfindungsgemäße künstliche neuronale Netz weist dementsprechend rückgekoppelte Neuronen auf. Insbesondere umfasst das dritte Teilnetz ein rekurrentes (rückgekoppeltes) neuronales Teilnetz. Damit ist das erfindungsgemäße künstliche neuronale Netz in der Lage, die zeitliche Entwicklung sowohl bei den radiologischen Aufnahmen als auch den Vitaldaten bei der Ermittlung des ARDS-Indikator-Wertes zu berücksichtigen.

Ein besonders geeignetes rekurrentes Netz ist ein Long Short-Term Memory (LSTM) oder ein Time-Aware LSTM Network (siehe beispielsweise I.M. Baytas et al.: Patient Subtyping via Time-Aware LSTM Networks, Proceedings of KDD '17, 2017, DOI:10.1145/3097983.3097997).

In einer bevorzugten Ausführungsform weist das dritte Teilnetz rückgekoppelte Neuronen auf. Das erste und/oder insbesondere das zweite Teilnetz können/kann ebenfalls rückgekoppelte Neuronen aufweisen.

In einer bevorzugten Ausführungsform weist das zweite Teilnetz einen Autoencoder auf und/oder es ist mit Hilfe eines Autoencoders vortrainiert worden. Das Ziel eines Autoencoders ist es, eine komprimierte Repräsentation (Encoding) für einen Satz Daten zu lernen und somit auch wesentliche Merkmale zu extrahieren. Dadurch kann er zur Dimensionsreduktion genutzt werden. Autoencoder sind beispielsweise beschrieben in Q. V. Le: A Tutorial on Deep Learning Part 2: Autoencoders, Convolutional Neural Networks and Recurrent Neural Networks, 2015, https://cs.stanford.edu/~quocle/tutorial2.pdf; W. Meng: Relational Autoencoder for Feature Extraction, arXiv:1802.03145v1 [cs.LG] 9 Feb 2018; WO2018046412A1).

In einer besonders bevorzugten Ausführungsform umfasst das zweite Teilnetz ein rekurrentes (rückgekoppeltes) neuronales Netz gefolgt von einem Autoencoder.

Die Erfindung wird nachfolgend anhand von Figuren näher erläutert, ohne die Erfindung auf die in den Figuren gezeigten Merkmale oder Merkmalskombinationen beschränken zu wollen.

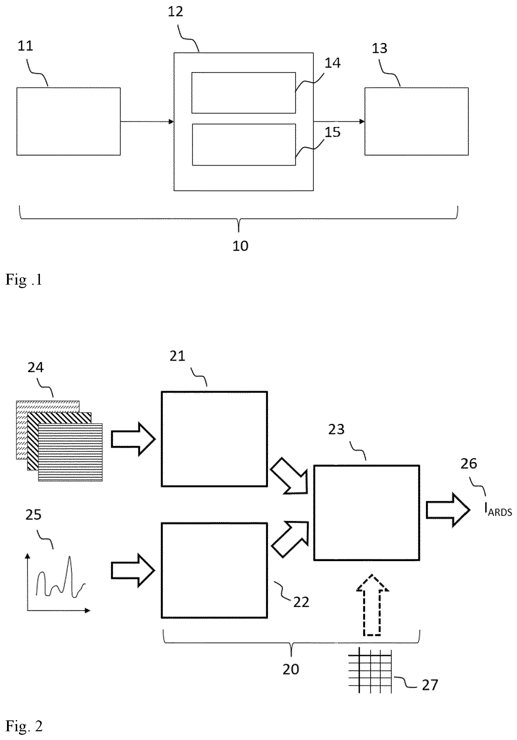

Fig. 1 zeigt schematisch eine Ausführungsform des erfindungsgemäßen Computersystems (10). Das Computersystem (10) umfasst eine Eingabeeinheit (11), eine Steuer- und Recheneinheit (12) und eine Ausgabeeinheit (13). Die Steuer- und Recheneinheit (12) umfasst eine Verarbeitungseinheit (*processing unit,* 14) mit einem oder mehreren Prozessoren zur Durchführung logischer Operationen und eine Speichereinheit (*memory,* 15)*.*

Über die Eingabeeinheit (11) werden Patientendaten empfangen und/oder abgerufen.

Die Verarbeitungseinheit (14) ist mit prozessorausführbaren Anweisungen (die in der Speichereinheit (15) gespeichert sein können) konfiguriert, anhand der Patientendaten mit Hilfe eines künstlichen neuronalen Netzes (das ebenfalls in der Speichereinheit (15) gespeichert sein kann), einen ARDS-Indikator-Wert zu ermitteln und diesen mit einem Schwellenwert zu vergleichen. Die Verarbeitungseinheit (14) ist ferner konfiguriert, bei einer definierten Abweichung des ARDS-Indikator-Werts von dem Schwellenwert eine Mitteilung über die Ausgabeeinheit (13) auszugeben.

Fig. 2 zeigt schematisch eine Ausführungsform des erfindungsgemäßen neuronalen Netzes (20). Das Netz (20) umfasst ein erstes Teilnetz (21), ein zweites Teilnetz (22) und ein drittes Teilnetz (23). Radiologische Aufnahmen (24) eines Intensivpatienten werden dem ersten Teilnetz (21) zugeführt. Vitaldaten (25) von Vitalparametern werden dem zweiten Teilnetz (22) zugeführt. Das erste Teilnetz (21) und das zweite Teilnetz (22) werden in dem dritten Teilnetz (23) zusammengeführt. Über das dritte Teilnetz (23) wird ein ARDS-Indikator-Wert (26) ausgegeben, der von dem neuronalen Netz (20) auf Basis der zugeführten Daten ermittelt worden ist.

Es ist denkbar, dass dem neuronalen Netz (20) weitere Daten (27) zugeführt werden, die bei der Ermittlung des ARDS-Indikator-Wertes berücksichtigt werden.

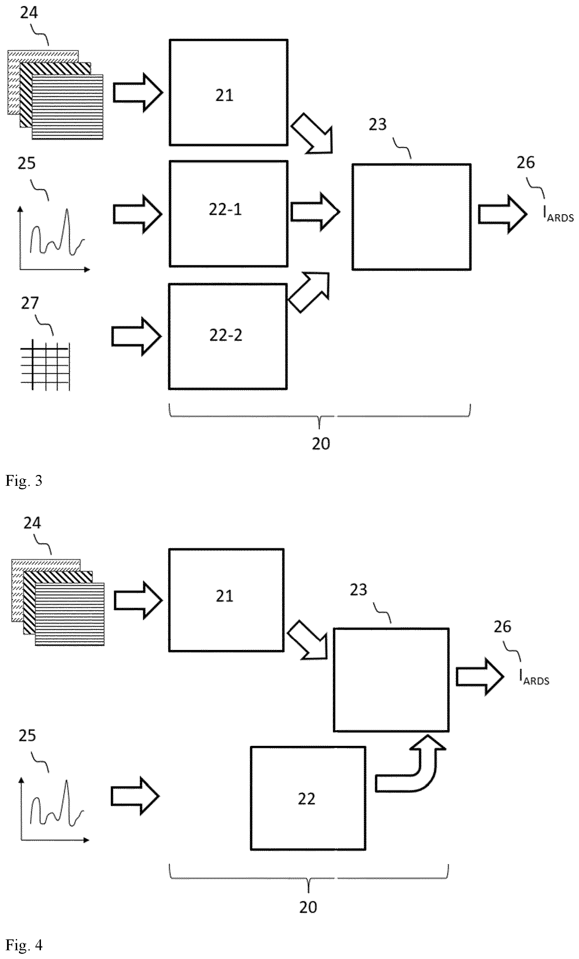

Fig. 3 zeigt schematisch eine weitere Ausführungsform des erfindungsgemäßen neuronalen Netzes (20). Das Netz (20) umfasst ein erstes Teilnetz (21), zwei zweite Teilnetze (22-1, 22-2) und ein drittes Teilnetz (23). Radiologische Aufnahmen (24) eines Intensivpatienten werden dem ersten Teilnetz (21) zugeführt. Vitaldaten (25) von Vitalparametern werden einem der zweiten Teilnetze (22-1) zugeführt. Weitere Patientendaten (27) werden dem anderen der zweiten Teilnetze (22-2) zugeführt. Das erste Teilnetz (21) und die zweiten Teilnetze (22-1, 22-2) werden in dem dritten Teilnetz (23) zusammengeführt. Über das dritte Teilnetz (23) wird ein ARDS-Indikator-Wert (26) ausgegeben, der von dem neuronalen Netz (20) auf Basis der zugeführten Daten ermittelt worden ist.

Fig. 4 zeigt schematisch eine weitere Ausführungsform des erfindungsgemäßen neuronalen Netzes (20). Das Netz (20) umfasst ein erstes Teilnetz (21), ein zweites Teilnetz (22) und ein drittes Teilnetz (23). Radiologische Aufnahmen (24) eines Intensivpatienten werden dem ersten Teilnetz (21) zugeführt. Vitaldaten (25) von Vitalparametern werden dem zweiten Teilnetz (22) zugeführt. Das erste Teilnetz (21) und das zweite Teilnetz (22) werden in dem dritten Teilnetz (23) zusammengeführt. Über das dritte Teilnetz (23) wird ein ARDS-Indikator-Wert (26) ausgegeben, der von dem neuronalen Netz (20) auf Basis der zugeführten Daten ermittelt worden ist.

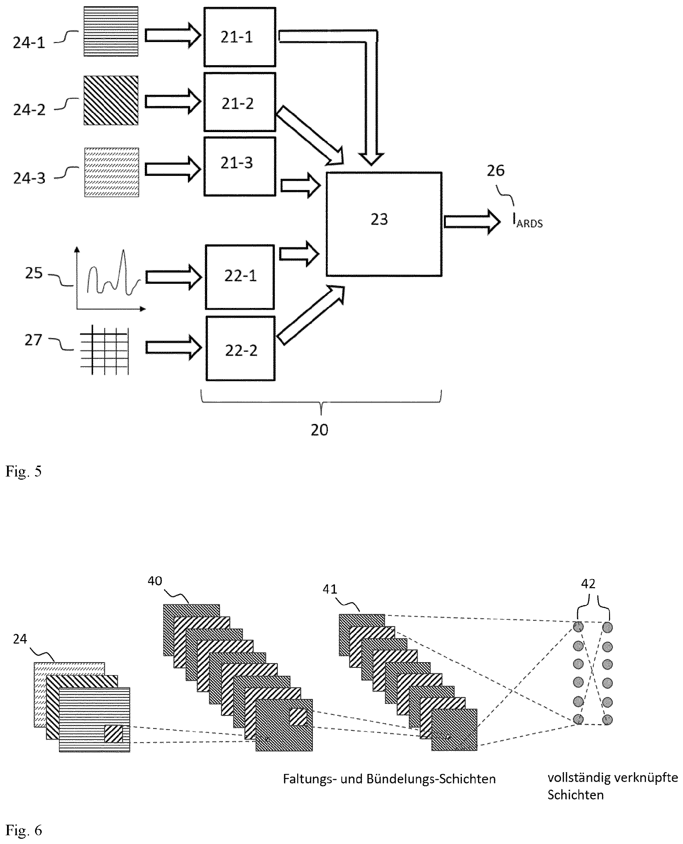

Fig. 5 zeigt schematisch eine weitere Ausführungsform des erfindungsgemäßen neuronalen Netzes (20). Im vorliegenden Beispiel sind mehrere erste Teilnetze (21-1, 21-2, 21-3) vorhanden, wobei jedes Teilnetz jeweils eine radiologische Aufnahme (24-1, 24-2, 24-3) verarbeitet. Die radiologischen Aufnahmen stammen vom selben Patienten, wurden aber vorzugsweise zu unterschiedlichen Zeitpunkten aufgenommen. Vorzugsweise sind die Strukturen und Gewichte der ersten Teilnetze identisch. Ferner sind mehrere zweite Teilnetze (22-1, 22-2) vorhanden, eines zur Verarbeitung von Vitaldaten (25) zu Vitalparametern, ein weiteres zur Verarbeitung von weiteren Daten (27). Vorzugsweise sind die Strukturen und/oder die Gewichte der zweiten Teilnetze nicht identisch. Die Teilnetze werden in einem dritten Teilnetz (23) zusammengeführt. Über das dritte Teilnetz (23) wird ein ARDS-Indikator-Wert (26) ausgegeben.

Fig. 6 zeigt beispielhaft und schematisch das Funktionsprinzip des ersten Teilnetzes zur Verarbeitung von radiologischen Aufnahmen, wobei das erste Teilnetz als ein CNN ausgeführt ist. In Fig. 4 sind verschiedene Schichten innerhalb eines CNN dargestellt. Radiologische Aufnahmen (24) werden dem CNN zugeführt. Beispielsweise können die Graustufen einer Rastergrafik pixel- oder voxelweise als Eingabedaten einer Eingabeschicht zugeführt werden. Das CNN umfasst üblicherweise eine Vielzahl an Faltungsschichten und Pooling-Schichten (40, 41). Innerhalb dieser Schichten werden Faltungsoperationen ausgeführt, deren Ausgabe an die nächste Schicht geleitet wird. Die Dimensionsreduzierung, die innerhalb der Faltungsschichten durchgeführt wird, ist ein Aspekt, der es dem CNN ermöglicht, große Bilder zu skalieren. Die Ausgabe der Faltungs- und Pooling-Schichten mündet üblicherweise in einer Mehrzahl an vollständig verbundene Schichten (*fully connected layers* 42).

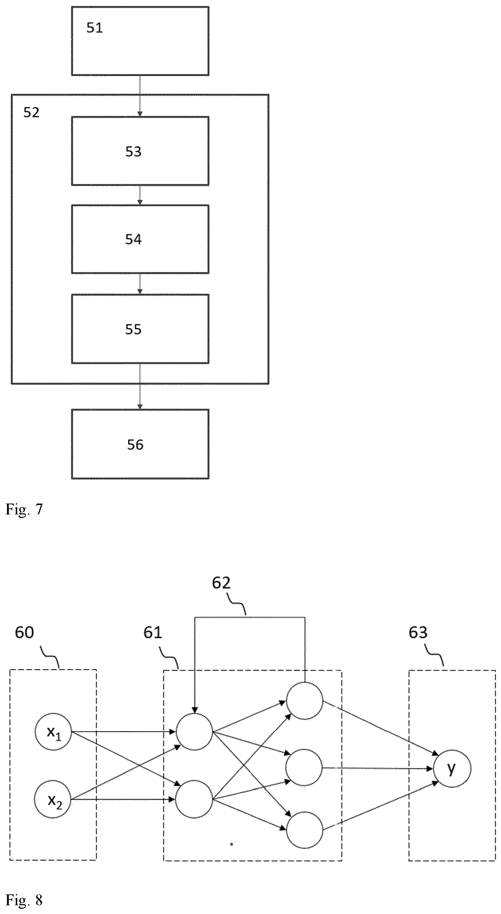

Fig. 7 zeigt beispielhaft die Berechnungsstufen innerhalb einer Faltungsschicht eines CNN. Die Eingabe (51) in eine Faltungsschicht (52) eines CNN kann in drei Stufen verarbeitet werden. Die drei Stufen können eine Faltungsstufe (53), eine Detektorstufe (54) und eine Sammelstufe (55) umfassen. Die Faltungsschicht (52) kann dann Daten an eine nachfolgende Faltungsschicht (56) ausgeben.

Fig. 8 zeigt beispielhaft ein rekurrentes neuronales Netzwerk. In einem rekurrenten neuronalen Netzwerk (RNN) beeinflusst der vorherige Status des Netzwerks die Ausgabe des aktuellen Status des Netzwerks. Das dargestellte RNN umfasst eine Eingabeschicht (60), die einen Eingabevektor (x₁, x₂) empfängt, versteckte Schichten (*hidden layer* 61) mit einem Rückkopplungsmechanismus (62) und eine Ausgabeschicht (63), um ein Ergebnis auszugeben.

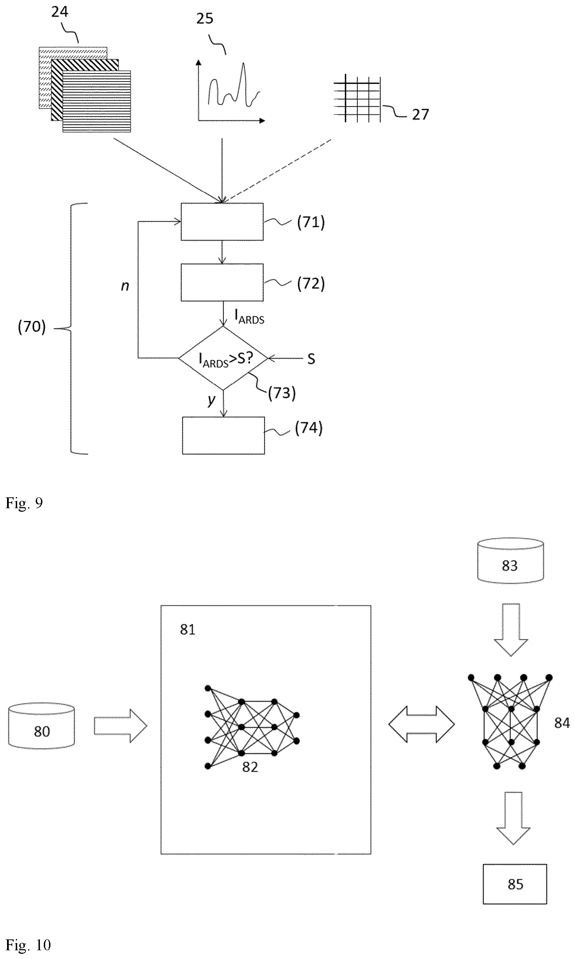

Fig. 9 zeigt schematisch in Form eines Ablaufdiagramms eine Ausführungsform des erfindungsgemäßen Verfahrens (70). In einem ersten Schritt (71) werden Patientendaten empfangen. Die Patientendaten umfassen eine Mehrzahl an radiologischen Aufnahmen (24) vom Thorax eines Intensivpatienten, eine Mehrzahl an Vitaldaten (25) zu Vitalparametern des Intensivpatienten und optional weitere Daten (27).

Die Patientendaten werden in einem weiteren Schritt (72) einem künstlichen neuronalen Netz zugeführt. Das künstliche neuronale Netz ist konfiguriert, auf Basis der Patientendaten einen ARDS-Indikator-Wert zu ermitteln. Dieser ARDS-Indikator-Wert wird in einem weiteren Schritt (73) mit einem Schwellenwert verglichen ("I_{ARDS} > S"?). Liegt eine definierte Abweichung zwischen dem ARDS-Indikator-Wert und dem Schwellenwert vor ("*y*"), wird in einem weiteren Schritt (74) eine Mitteilung ausgegeben, die darauf hinweist, dass die Wahrscheinlichkeit groß ist, dass beim Intensivpatienten ARDS auftritt. Ist die Wahrscheinlichkeit, dass ARDS vorliegt, hingegen gering ("*n*"), erfolgt keine Mitteilung; stattdessen wird das erfindungsgemäße Verfahren (70) erneut durchlaufen, sobald neue Patientendaten vorliegen.

Fig. 10 zeigt beispielhaft ein Training eines künstlichen neuronalen Netzwerks. Sobald ein gegebenes Netzwerk (82) für eine Aufgabe strukturiert wurde, wird das neuronale Netzwerk unter Verwendung eines Trainingsdatensatzes (80) trainiert. Um den Trainingsprozess zu starten, können die Anfangsgewichte zufällig oder durch Vortraining z.B. unter Verwendung eines Deep-Believe-Netzwerks ausgewählt werden. Der Trainingszyklus kann dann entweder überwacht oder unbeaufsichtigt durchgeführt werden. Überwachtes Lernen ist eine Lernmethode, bei der das Training als vermittelte Operation ausgeführt wird, beispielsweise wenn der Trainingsdatensatz (1102) eine Eingabe gepaart mit der gewünschten Ausgabe für die Eingabe enthält oder wenn der Trainingsdatensatz eine Eingabe mit bekannter Ausgabe enthält. Das Netzwerk verarbeitet die Eingaben und vergleicht die resultierenden Ausgaben mit einem Satz erwarteter oder gewünschter Ausgaben. Die Gewichte werden dann so verändert, dass der Fehler minimiert wird. Der Trainingsrahmen (81) kann Werkzeuge bereitstellen, um zu überwachen, wie gut das nicht trainierte neuronale Netzwerk (82) in Richtung eines Modells konvergiert, das geeignet ist, korrekte Antworten basierend auf bekannten Eingabedaten zu generieren. Der Trainingsprozess kann fortgesetzt werden, bis das neuronale Netzwerk eine statistisch gewünschte Genauigkeit erreicht. Das trainierte neuronale Netzwerk (84) kann dann eingesetzt werden, um eine Ausgabe für neue Daten zu erzeugen.

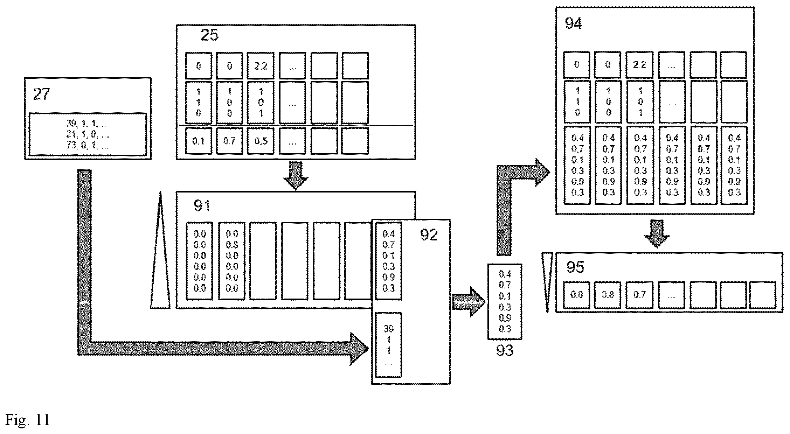

Fig. 11 zeigt beispielhaft und schematisch eine Möglichkeit der Verarbeitung von Vitaldaten (25) und weiteren Daten (27) mittels eines künstlichen neuronalen Netzes. Das künstliche neuronale Netz umfasst ein Teilnetz (91) mit einer Mehrzahl an rückgekoppelten Schichten zur Verarbeitung der Zeitinformationen in den Vitaldaten (25). Die verarbeiteten Vitaldaten werden mit den weiteren Daten (27) in einem Teilnetz (92) mit einer Mehrzahl an verbundenen Schichten (*fully connected layers*) zusammengeführt. Die zusammengeführten Daten werden anschließend einem Engpass (93) mit einer definierten Größe zugeführt, um eine Dimensionsreduzierung zu erreichen. Anschließend werden die Daten rekonstruiert (94). Es schließt sich wiederum ein Teilnetz (95) mit einer Mehrzahl an rückgekoppelten Schichten an, um eine Repräsentation der ursprünglichen Daten (25, 27) zu erzeugen. Der Aufbau der Teilnetze (91) -> (92) -> (93) -> (94) -> (95) entspricht einem Autoencoder.

## Patentansprüche

1. Computersystem (10) umfassend
- eine Eingabeeinheit (11),
- eine Steuer- und Recheneinheit (12) und
- eine Ausgabeeinheit (13)
wobei die Steuer- und Recheneinheit (12) konfiguriert ist, die Eingabeeinheit (11) zu veranlassen, Patientendaten zu einem Intensivpatienten zu empfangen, wobei die Patientendaten mindestens die folgenden Patientendaten umfassen:
∘ eine Mehrzahl an radiologischen Aufnahmen (24) vom Thorax des Intensivpatienten, wobei die radiologischen Aufnahmen (24) den Thorax zu unterschiedlichen Zeitpunkten zeigen, und
∘ eine Mehrzahl an Vitaldaten (25) zu Vitalparametern des Intensivpatienten, wobei die Vitaldaten (25) Werte zu den Vitalparametern zu unterschiedlichen Zeitpunkten angeben,
wobei die Steuer- und Recheneinheit (12) konfiguriert ist, die empfangenen Patientendaten einem künstlichen neuronalen Netz (20) zuzuführen,
∘ wobei das künstliche neuronale Netz mindestens (20) drei Teilnetze umfasst, ein erstes Teilnetz (21), ein zweites Teilnetz (22) und ein drittes Teilnetz (23),
∘ wobei das erste Teilnetz (21) eine erste Eingabeschicht umfasst, wobei das zweite Teilnetz (22) eine zweite Eingabeschicht umfasst, wobei das dritte Teilnetz (23) eine Ausgabeschicht umfasst, und wobei das erste Teilnetz (21) und das zweite Teilnetz (22) in dem dritten Teilnetz (23) zusammengeführt werden,
∘ wobei die Mehrzahl an radiologischen Aufnahmen (24) der ersten Eingabeschicht zugeführt wird und die Mehrzahl an Vitaldaten (25) der zweiten Eingabeschicht zugeführt wird,
∘ wobei das erste Teilnetz (21) konfiguriert ist, für jede radiologische Aufnahme (24) einen zeitabhängigen Bild-Deskriptor zu erzeugen,
∘ wobei das zweite Teilnetz (22) konfiguriert ist, aus den Vitaldaten (25) zeitabhängige Vitaldaten-Deskriptoren zu erzeugen,
∘ wobei die zeitabhängigen Bild-Deskriptoren und die zeitabhängigen Vitaldaten-Deskriptoren Schichten in dem künstlichen neuronalen Netz (20) zugeführt werden, die rückgekoppelte Neuronen umfassen,
∘ wobei das künstliche neuronale Netz (20) mittels Referenzdaten trainiert worden ist, anhand von Patientendaten einen ARDS-Indikator-Wert (26) zu berechnen und den ARDS-Indikator-Wert (26) über die Ausgangsschicht auszugeben,
wobei die Steuer- und Recheneinheit (12) konfiguriert ist, den ARDS-Indikator-Wert (26) von dem künstlichen neuronalen Netz (20) zu empfangen,
wobei die Steuer- und Recheneinheit (12) konfiguriert ist, den ARDS-Indikator-Wert (26) mit einem Schwellenwert zu vergleichen, und
wobei die Steuer- und Recheneinheit (12) konfiguriert ist, die Ausgabeeinheit (13) zu veranlassen, eine Mitteilung auszugeben, wenn der ARDS-Indikator-Wert (26) in definierter Weise von dem Schwellenwert abweicht.

2. Computersystem (10) gemäß Anspruch 1, wobei die Mehrzahl an radiologischen Aufnahmen (24) mindestens drei Röntgenbilder des Thorax des Intensivpatienten umfassen, wobei mindestens ein Röntgenbild innerhalb der letzten zwölf Stunden, vorzugsweise innerhalb der letzten drei Stunden erzeugt worden ist.

3. Computersystem (10) gemäß einem der Ansprüche 1 oder 2, wobei die Vitalparameter (25) ausgewählt sind aus der Reihe: Herzfrequenz, Atemfrequenz, Blutdruck, Körpertemperatur, Sauerstoffsättigung im Blut, Sauerstoffpartialdruck, inspiratorische Sauerstofffraktion, Oxygenierungsindex und/oder pH-Wert des Bluts des Intensivpatienten.

4. Computersystem (10) gemäß einem der Ansprüche 1 bis 3, wobei die Steuer- und Recheneinheit (12) konfiguriert ist, die Eingabeeinheit (11) zu veranlassen, weitere Patientendaten (27) zu einem Intensivpatienten zu empfangen, wobei die weiteren Patientendaten (27) ausgewählt sind aus der Reihe: Alter, Geschlecht, Körpergewicht, Körpergröße, vorliegende Erkrankung(en) und/oder frühere Erkrankung(en) des Intensivpatienten, wobei die Steuer- und Recheneinheit (12) konfiguriert ist, die empfangenen Patientendaten einem künstlichen neuronalen Netz (20) zuzuführen, wobei das künstliche neuronale Netz (20) ein erstes Teilnetz (21), zwei zweite Teilnetze (22-1, 22-2) und ein drittes Teilnetz (23) umfasst, wobei das erste Teilnetz (21) eine erste Eingabeschicht umfasst, wobei das eine der zweiten Teilnetze (22-1) eine zweite Eingabeschicht umfasst, wobei das andere der zweiten Teilnetze (22-2) eine dritte Eingabeschicht umfasst, wobei das dritte Teilnetz (23) eine Ausgabeschicht umfasst, und wobei das erste Teilnetz (21) und die zweiten Teilnetze (22-1, 22-2) in dem dritten Teilnetz (23) zusammengeführt werden, wobei die weiteren Patientendaten (27) der dritten Eingabeschicht zugeführt werden.

5. Computersystem (10) gemäß einem der Ansprüche 1 bis 4, wobei die Mitteilung Handlungsempfehlungen umfasst, die einen Arzt oder Krankenhauspersonal ergreifen sollen, um eine Verschlechterung des Gesundheitszustands des Intensivpatienten zu verhindern.

6. Computersystem (10) gemäß einem der Ansprüche 1 bis 5, wobei das Computersystem (10) ferner konfiguriert ist, den Gesundheitszustand des Intensivpatienten auf einer Intensivstation eines Krankenhauses anhand der Vitalparamater zu überwachen.

7. Computersystem (10) gemäß einem der Ansprüche 1 bis 6, wobei das Computersystem (10) auf mindestens eine Datenbank eines Krankenhauses zugreifen kann, in der ein Teil der Patientendaten gespeichert sind.

8. Computersystem (10) gemäß einem der Ansprüche 1 bis 7, wobei das Computersystem (10) konfiguriert ist, einen neuen ARDS-Indikator-Wert (26) immer dann zu berechnen, wenn neue definierte Patientendaten vorliegen.

9. Computersystem (10) gemäß einem der Ansprüche 1 bis 8, wobei das erste Teilnetz (21) ein CNN ist oder ein CNN umfasst und/oder wobei das dritte Teilnetz (23) ein RNN ist oder ein RNN umfasst.

10. Computersystem (10) gemäß einem der Ansprüche 1 bis 9, wobei das zweite Teilnetz (22) ein RNN gefolgt von einem Autoencoder ist.

11. Verfahren zur Erkennung von ARDS bei einem Intensivpatienten umfassend die Schritte
- Empfangen von Patientendaten zu dem Intensivpatienten, wobei die Patientendaten mindestens die folgenden Patientendaten umfassen:
∘ eine Mehrzahl an radiologischen Aufnahmen (24) vom Thorax des Intensivpatienten, wobei die radiologischen Aufnahmen (24) den Thorax zu unterschiedlichen Zeitpunkten zeigen, und
∘ eine Mehrzahl an Vitaldaten (25) zu Vitalparametern des Intensivpatienten, wobei die Vitaldaten (25) Werte zu den Vitalparametern zu unterschiedlichen Zeitpunkten angeben,
- Zuführen der Patientendaten einem künstlichen neuronalen Netz (20),
∘ wobei das künstliche neuronale Netz (20) mindestens drei Teilnetze umfasst, ein erstes Teilnetz (21), ein zweites Teilnetz (22) und ein drittes Teilnetz (23),
∘ wobei das erste Teilnetz (21) eine erste Eingabeschicht umfasst, wobei das zweite Teilnetz (22) eine zweite Eingabeschicht umfasst, wobei das dritte Teilnetz (23) eine Ausgabeschicht umfasst, und wobei das erste Teilnetz (21) und das zweite Teilnetz (22) in dem dritten Teilnetz (23) zusammengeführt werden,
∘ wobei die Mehrzahl an radiologischen Aufnahmen (24) der ersten Eingabeschicht zugeführt wird und die Mehrzahl an Vitaldaten (25) der zweiten Eingabeschicht zugeführt wird,
∘ wobei das erste Teilnetz (21) konfiguriert ist, für jede radiologische Aufnahme (24) einen zeitabhängigen Bild-Deskriptor zu erzeugen,
∘ wobei das zweite Teilnetz (22) konfiguriert ist, aus den Vitaldaten (25) zeitabhängige Vitaldaten-Deskriptoren zu erzeugen,
∘ wobei die zeitabhängigen Bild-Deskriptoren und die zeitabhängigen Vitaldaten-Deskriptoren Schichten in dem künstlichen neuronalen Netz (20) zugeführt werden, die rückgekoppelte Neuronen umfassen,
∘ wobei das künstliche neuronale Netz (20) mittels Referenzdaten trainiert worden ist, anhand von Patientendaten einen ARDS-Indikator-Wert (26) zu berechnen und den ARDS-Indikator-Wert (26) über die Ausgangsschicht auszugeben,
- Empfangen eines ARDS-Indikator-Wertes (26) für die zugeführten Patientendaten von dem künstlichen neuronalen Netz (20),
- Vergleichen des ARDS-Indikator-Werts (26) mit einem Schwellenwert,
- Ausgeben einer Mitteilung für den Fall, dass der ARDS-Indikator-Wert (26) in definierter Weise von dem Schwellenwert abweicht.

12. Computerprogrammprodukt umfassend ein Computerprogramm, das in einen Arbeitsspeicher eines Computersystems (10) geladen werden kann und dort das Computersystem (10) dazu veranlasst, folgende Schritte ausführen:
- Empfangen von Patientendaten zu dem Intensivpatienten, wobei die Patientendaten mindestens die folgenden Patientendaten umfassen:
∘ eine Mehrzahl an radiologischen Aufnahmen (24) vom Thorax des Intensivpatienten, wobei die radiologischen Aufnahmen (24) den Thorax zu unterschiedlichen Zeitpunkten zeigen, und
∘ eine Mehrzahl an Vitaldaten (25) zu Vitalparametern des Intensivpatienten, wobei die Vitaldaten (25) Werte zu den Vitalparametern zu unterschiedlichen Zeitpunkten angeben,
- Zuführen der Patientendaten einem künstlichen neuronalen Netz (20),
∘ wobei das künstliche neuronale Netz (20) mindestens drei Teilnetze umfasst, ein erstes Teilnetz (21), ein zweites Teilnetz (22) und ein drittes Teilnetz (23),
∘ wobei das erste Teilnetz (21) eine erste Eingabeschicht umfasst, wobei das zweite Teilnetz (22) eine zweite Eingabeschicht umfasst, wobei das dritte Teilnetz (23) eine Ausgabeschicht umfasst, und wobei das erste Teilnetz (21) und das zweite Teilnetz (22) in dem dritten Teilnetz (23) zusammengeführt werden,
∘ wobei die Mehrzahl an radiologischen Aufnahmen (24) der ersten Eingabeschicht zugeführt wird und die Mehrzahl an Vitaldaten (25) der zweiten Eingabeschicht zugeführt wird,
∘ wobei das erste Teilnetz (21) konfiguriert ist, für jede radiologische Aufnahme (24) einen zeitabhängigen Bild-Deskriptor zu erzeugen,
∘ wobei das zweite Teilnetz (22) konfiguriert ist, aus den Vitaldaten (25) zeitabhängige Vitaldaten-Deskriptoren zu erzeugen,
∘ wobei die zeitabhängigen Bild-Deskriptoren und die zeitabhängigen Vitaldaten-Deskriptoren Schichten in dem künstlichen neuronalen Netz (20) zugeführt werden, die rückgekoppelte Neuronen umfassen,
∘ wobei das künstliche neuronale Netz (22) mittels Referenzdaten trainiert worden ist, anhand von Patientendaten einen ARDS-Indikator-Wert (26) zu berechnen und den ARDS-Indikator-Wert (26) über die Ausgangsschicht auszugeben,
- Empfangen eines ARDS-Indikator-Wertes (26) für die zugeführten Patientendaten von dem künstlichen neuronalen Netz (20),
- Vergleichen des ARDS-Indikator-Werts (26) mit einem definierten Schwellenwert,
- Ausgeben einer Mitteilung für den Fall, dass der ARDS-Indikator-Wert (26) in definierter Weise von dem Schwellenwert abweicht.

## Claims

1. Computer system (10) comprising
- an input unit (11),
- a control and calculation unit (12) and
- an output unit (13)
wherein the control and calculation unit (12) is configured to cause the input unit (11) to receive patient data relating to an intensive care patient,
wherein the patient data comprise at least the following patient data:
∘ a plurality of radiological images (24) of the thorax of the intensive care patient, wherein the radiological images (24) show the thorax at different times, and
∘ a plurality of vital data (25) relating to vital parameters of the intensive care patient, wherein the vital data (25) specify values relating to the vital parameters at different times,
wherein the control and calculation unit (12) is configured to supply the received patient data to an artificial neural network (20),
∘ wherein the artificial neural network (20) comprises at least three subnetworks, a first subnetwork (21), a second subnetwork (22) and a third subnetwork (23),
∘ wherein the first subnetwork (21) comprises a first input layer, wherein the second subnetwork (22) comprises a second input layer, wherein the third subnetwork (23) comprises an output layer, and wherein the first subnetwork (21) and the second subnetwork (22) are merged in the third subnetwork (23),
∘ wherein the plurality of radiological images (24) is supplied to the first input layer and the plurality of vital data (25) is supplied to the second input layer,
∘ wherein the first subnetwork (21) is configured to generate a time-dependent image descriptor for each radiological image (24),
∘ wherein the second subnetwork (22) is configured to generate time-dependent vital data descriptors from the vital data (25),
∘ wherein the time-dependent image descriptors and the time-dependent vital data descriptors are supplied to layers in the artificial neural network (20) that comprise feedback neurons,
∘ wherein the artificial neural network (20) has been trained using reference data to calculate an ARDS indicator value (26) on the basis of patient data and to output the ARDS indicator value (26) via the output layer,
wherein the control and calculation unit (12) is configured to receive the ARDS indicator value (26) from the artificial neural network (20),
wherein the control and calculation unit (12) is configured to compare the ARDS indicator value (26) with a threshold value, and
wherein the control and calculation unit (12) is configured to cause the output unit (13) to output a notification if the ARDS indicator value (26) deviates from the threshold value in a defined manner.

2. Computer system (10) according to Claim 1, wherein the plurality of radiological images (24) comprise at least three X-rays of the thorax of the intensive care patient, wherein at least one X-ray has been generated within the last twelve hours, preferably within the last three hours.

3. Computer system (10) according to either of Claims 1 and 2, wherein the vital parameters (25) are selected from the group: heart rate, respiratory rate, blood pressure, body temperature, blood oxygen saturation, partial pressure of oxygen, fraction of inspired oxygen, oxygenation index and/or blood pH of the intensive care patient.

4. Computer system (10) according to any of Claims 1 to 3, wherein the control and calculation unit (12) is configured to cause the input unit (11) to receive further patient data (27) relating to an intensive care patient, wherein the further patient data (27) are selected from the group: age, sex, body weight, height, existing disease (s) and/or previous disease(s) of the intensive care patient, wherein the control and calculation unit (12) is configured to supply the received patient data to an artificial neural network (20), wherein the artificial neural network (20) comprises a first subnetwork (21), two second subnetworks (22-1, 22-2) and a third subnetwork (23), wherein the first subnetwork (21) comprises a first input layer, wherein one of the second subnetworks (22-1) comprises a second input layer, wherein the other of the second subnetworks (22-2) comprises a third input layer, wherein the third subnetwork (23) comprises an output layer, and wherein the first subnetwork (21) and the second subnetworks (22-1, 22-2) are merged in the third subnetwork (23), wherein the further patient data (27) are supplied to the third input layer.

5. Computer system (10) according to any of Claims 1 to 4, wherein the notification comprises recommended actions to be taken by a physician or hospital staff in order to prevent deterioration of the state of health of the intensive care patient.

6. Computer system (10) according to any of Claims 1 to 5, wherein the computer system (10) is further configured to monitor the state of health of the intensive care patient in an intensive care unit of a hospital on the basis of the vital parameters.

7. Computer system (10) according to any of Claims 1 to 6, wherein the computer system (10) can access at least one database of a hospital in which some of the patient data are stored.

8. Computer system (10) according to any of Claims 1 to 7, wherein the computer system (10) is configured to calculate a new ARDS indicator value (26) whenever new defined patient data are available.

9. Computer system (10) according to any of Claims 1 to 8, wherein the first subnetwork (21) is a CNN or comprises a CNN and/or wherein the third subnetwork (23) is an RNN or comprises an RNN.

10. Computer system (10) according to any of Claims 1 to 9, wherein the second subnetwork (22) is an RNN followed by an autoencoder.

11. Computer-implemented method for detecting ARDS in an intensive care patient, comprising the steps of
- receiving patient data relating to the intensive care patient, wherein the patient data comprise at least the following patient data:
∘ a plurality of radiological images (24) of the thorax of the intensive care patient, wherein the radiological images (24) show the thorax at different times, and
∘ a plurality of vital data (25) relating to vital parameters of the intensive care patient, wherein the vital data (25) specify values relating to the vital parameters at different times,
- supplying the patient data to an artificial neural network (20),
∘ wherein the artificial neural network (20) comprises at least three subnetworks, a first subnetwork (21), a second subnetwork (22) and a third subnetwork (23),
∘ wherein the first subnetwork (21) comprises a first input layer, wherein the second subnetwork (22) comprises a second input layer, wherein the third subnetwork (23) comprises an output layer, and wherein the first subnetwork (21) and the second subnetwork (22) are merged in the third subnetwork (23),
∘ wherein the plurality of radiological images (24) is supplied to the first input layer and the plurality of vital data (25) is supplied to the second input layer,
∘ wherein the first subnetwork (21) is configured to generate a time-dependent image descriptor for each radiological image (24),
∘ wherein the second subnetwork (22) is configured to generate time-dependent vital data descriptors from the vital data (25),
∘ wherein the time-dependent image descriptors and the time-dependent vital data descriptors are supplied to layers in the artificial neural network (20) that comprise feedback neurons,
∘ wherein the artificial neural network (20) has been trained using reference data to calculate an ARDS indicator value (26) on the basis of patient data and to output the ARDS indicator value (26) via the output layer,
- receiving an ARDS indicator value (26) for the supplied patient data from the artificial neural network (20),
- comparing the ARDS indicator value (26) with a threshold value,
- outputting a notification if the ARDS indicator value (26) deviates from the threshold value in a defined manner.

12. Computer program product comprising a computer program that can be loaded into a working memory of a computer system (10), where it causes the computer system (10) to execute the following steps:
- receiving patient data relating to the intensive care patient, wherein the patient data comprise at least the following patient data:
∘ a plurality of radiological images (24) of the thorax of the intensive care patient, wherein the radiological images (24) show the thorax at different times, and
∘ a plurality of vital data (25) relating to vital parameters of the intensive care patient, wherein the vital data (25) specify values relating to the vital parameters at different times,
- supplying the patient data to an artificial neural network (20),
∘ wherein the artificial neural network (20) comprises at least three subnetworks, a first subnetwork (21), a second subnetwork (22) and a third subnetwork (23),
∘ wherein the first subnetwork (21) comprises a first input layer, wherein the second subnetwork (22) comprises a second input layer, wherein the third subnetwork (23) comprises an output layer, and wherein the first subnetwork (21) and the second subnetwork (22) are merged in the third subnetwork (23),
∘ wherein the plurality of radiological images (24) is supplied to the first input layer and the plurality of vital data (25) is supplied to the second input layer,
∘ wherein the first subnetwork (21) is configured to generate a time-dependent image descriptor for each radiological image (24),
∘ wherein the second subnetwork (22) is configured to generate time-dependent vital data descriptors from the vital data (25),
∘ wherein the time-dependent image descriptors and the time-dependent vital data descriptors are supplied to layers in the artificial neural network (20) that comprise feedback neurons,
∘ wherein the artificial neural network (22) has been trained using reference data to calculate an ARDS indicator value (26) on the basis of patient data and to output the ARDS indicator value (26) via the output layer,
- receiving an ARDS indicator value (26) for the supplied patient data from the artificial neural network (20),
- comparing the ARDS indicator value (26) with a defined threshold value,
- outputting a notification if the ARDS indicator value (26) deviates from the threshold value in a defined manner.

## Revendications

1. Système informatique (10) comprenant
- une unité d'entrée (11),
- une unité de commande et de calcul (12), et
- une unité de sortie (13),
l'unité de commande et de calcul (12) étant configurée pour amener l'unité d'entrée (11) à recevoir des données de patient concernant un patient en soins intensifs, les données de patient comprenant au moins les données de patient suivantes :
∘ une multitude de clichés radiologiques (24) du thorax du patient en soins intensifs, les clichés radiologiques (24) montrant le thorax à des moments différents, et
∘ une multitude de données vitales (25) concernant des paramètres vitaux du patient en soins intensifs, les données vitales (25) indiquant des valeurs concernant des paramètres vitaux à des moments différents,
l'unité de commande et de calcul (12) étant configurée pour amener les données de patient reçues à un réseau neuronal artificiel (20),
∘ le réseau neuronal artificiel (20) comprenant au moins trois réseaux partiels, un premier réseau partiel (21), un deuxième réseau partiel (22) et un troisième réseau partiel (23),
∘ le premier réseau partiel (21) comprenant une première couche d'entrée, le deuxième réseau partiel (22) comprenant une deuxième couche d'entrée, le troisième réseau partiel (23) comprenant une couche d'envoi, et le premier réseau partiel (21) et le deuxième réseau partiel (22) étant regroupés dans le troisième réseau partiel (23),
∘ la multitude de clichés radiologiques (24) étant amenée à la première couche d'entrée et la multitude de données vitales (25) étant amenée à la deuxième couche d'entrée,
∘ le premier réseau partiel (21) étant configuré pour générer pour chaque cliché radiologique (24) un descripteur d'image dépendant du temps,
∘ le deuxième réseau partiel (22) étant configuré pour générer à partir des données vitales (25) des descripteurs de données vitales dépendant du temps,
∘ les descripteurs d'image dépendant du temps et les descripteurs de données vitales dépendant du temps étant amenés dans le réseau neuronal artificiel (20), qui comprennent des neurones rétroactifs,
∘ le réseau neuronal artificiel (20) ayant été entraîné au moyen de données de référence pour calculer à l'aide de données de patient une valeur indiquant un SDRA (26) et pour envoyer la valeur indiquant un SDRA (26) par la couche de sortie,
l'unité de commande et de calcul (12) étant configurée pour recevoir du réseau neuronal artificiel (20) la valeur indiquant un SDRA (26),
l'unité de commande et de calcul (12) étant configurée pour comparer la valeur indiquant un SDRA (26) à une valeur de seuil, et
l'unité de commande et de calcul (12) étant configurée pour amener l'unité d'envoi (13) à envoyer une notification lorsque la valeur indiquant un SDRA (26) diffère de la valeur de seuil d'une manière définie.

2. Système informatique (10) selon la revendication 1, la multitude de clichés radiologiques (24) comprenant au moins trois radiographies du thorax du patient en soins intensifs, au moins une radiographie ayant été générée dans les dernières douze heures, de préférence dans les trois dernières heures.

3. Système informatique (10) selon l'une des revendications 1 ou 2, les paramètres vitaux (25) étant choisis parmi la série : fréquence cardiaque, fréquence respiratoire, tension artérielle, température corporelle, saturation en oxygène dans le sang, pression partielle d'oxygène, fraction d'oxygène lors de l'inspiration, indice d'oxygénation et/ou valeur de pH du sang du patient en soins intensifs.

4. Système informatique (10) selon l'une des revendications 1 à 3, l'unité de commande et de calcul (12) étant configurée pour amener l'unité d'entrée (11) à recevoir des données de patient supplémentaires (27) concernant un patient en soins intensifs, les données de patient supplémentaires (27) étant choisies dans la série : âge, sexe, poids, taille, maladie(s) existante(e) et/ou maladie(s) antérieure(s) du patient en soins intensifs, l'unité de commande et de calcul (12) étant configurée pour amener à un réseau neuronal artificiel (20) les données de patient reçues, le réseau neuronal artificiel (20) comprenant un premier réseau partiel (21), deux deuxièmes réseaux partiels (22-1, 22-2) et un troisième réseau partiel (23), le premier réseau partiel (21) comprenant une première couche d'entrée, un des deuxièmes réseaux partiels (22-1) comprenant une deuxième couche d'entrée, l'autre des deuxièmes réseaux partiels (22-2) comprenant une troisième couche d'entrée, le troisième réseau partiel (23) comprenant une couche d'envoi, et le premier réseau partiel (21) et les deuxièmes réseaux partiels (22-1, 22-2) étant regroupés en le troisième réseau partiel (23), les données de patient supplémentaires (27) étant amenées à la troisième couche d'entrée.

5. Système informatique (10) selon l'une des revendications 1 à 4, la notification comprenant des recommandations que le médecin ou le personnel hospitalier doit prendre pour empêcher une aggravation de l'état de santé du patient en soins intensifs.

6. Système informatique (10) selon l'une des revendications 1 à 5, le système informatique (10) étant configuré en outre pour surveiller l'état de santé du patient en soins intensifs dans une unité de soins intensifs d'un hôpital à l'aide des paramètres vitaux.

7. Système informatique (10) selon l'une des revendications 1 à 6, le système informatique (10) pouvant recourir à au moins une base de données d'un hôpital, dans laquelle une partie des données de patient sont stockées.

8. Système informatique (10) selon l'une des revendications 1 à 7, le système informatique (10) étant configuré pour toujours calculer une nouvelle valeur indiquant un SDRA (26) lorsque de nouvelles données de patient définies sont présentes.

9. Système informatique (10) selon l'une des revendications 1 à 8, le premier réseau partiel (21) étant un réseau de neurones convolutifs (CNN) ou comprenant un réseau de neurones convolutifs et/ou le troisième réseau partiel (23) étant un réseau de neurones récurrents (RNN) ou comprenant un réseau de neurones récurrents (RNN).

10. Système informatique (10) selon l'une des revendications 1 à 9, le deuxième réseau partiel (22) étant un réseau de neurones récurrents (RNN) suivi d'un autocodeur.

11. Procédé mis en œuvre par ordinateur destiné à identifier un SDRA chez un patient en soins intensifs, comprenant les étapes
- réception de données de patient concernant le patient en soins intensifs, les données de patient comprenant au moins les données de patient suivantes :
∘ une multitude de clichés radiologiques (24) du thorax du patient en soins intensifs, les clichés radiologiques (24) montrant le thorax à des moments différents, et
∘ une multitude de données vitales (25) concernant des paramètres vitaux du patient en soins intensifs, les données vitales (25) indiquant des valeurs concernant des paramètres vitaux à des moments différents,
- amenée des données de patient à un réseau neuronal artificiel (20),
∘ le réseau neuronal artificiel (20) comprenant au moins trois réseaux partiels, un premier réseau partiel (21), un deuxième réseau partiel (22) et un troisième réseau partiel (23),
∘ le premier réseau partiel (21) comprenant une première couche d'entrée, le deuxième réseau partiel (22) comprenant une deuxième couche d'entrée, le troisième réseau partiel (23) comprenant une couche d'envoi, et le premier réseau partiel (21) et le deuxième réseau partiel (22) étant regroupés dans le troisième réseau partiel (23),
∘ la multitude de clichés radiologiques (24) étant amenée à la première couche d'entrée et la multitude de données vitales (25) étant amenée à la deuxième couche d'entrée,
∘ le premier réseau partiel (21) étant configuré pour générer pour chaque cliché radiologique (24) un descripteur d'image dépendant du temps,
∘ le deuxième réseau partiel (22) étant configuré pour générer à partir des données vitales (25) des descripteurs de données vitales dépendant du temps,
∘ les descripteurs d'image dépendant du temps et les descripteurs de données vitales dépendant du temps étant amenés dans le réseau neuronal artificiel (20), qui comprennent des neurones rétroactifs,
∘ le réseau neuronal artificiel (20) ayant été entraîné au moyen de données de référence pour calculer à l'aide de données de patient une valeur indiquant un SDRA (26) et pour envoyer la valeur indiquant un SDRA (26) par la couche de sortie,
- réception d'une valeur indiquant un SDRA (26) pour les données de patient amenées par le réseau neuronal artificiel (20),
- comparaison de la valeur indiquant un SDRA (26) à une valeur de seuil,
- envoi d'une notification si la valeur indiquant un SDRA (26) diffère de la valeur de seuil d'une manière définie.

12. Produit-programme d'ordinateur comprenant un programme informatique, qui peut être chargé dans une mémoire de travail d'un système informatique (10) et qui, dans celui-ci, amène le système informatique (10) à exécuter les étapes suivantes :
- réception de données de patient concernant le patient en soins intensifs, les données de patient comprenant au moins les données de patient suivantes :
∘ une multitude de clichés radiologiques (24) du thorax du patient en soins intensifs, les clichés radiologiques (24) montrant le thorax à des moments différents, et
∘ une multitude de données vitales (25) concernant des paramètres vitaux du patient en soins intensifs, les données vitales (25) indiquant des valeurs concernant des paramètres vitaux à des moments différents,
- amenée des données de patient à un réseau neuronal artificiel (20),
∘ le réseau neuronal artificiel (20) comprenant au moins trois réseaux partiels, un premier réseau partiel (21), un deuxième réseau partiel (22) et un troisième réseau partiel (23),
∘ le premier réseau partiel (21) comprenant une première couche d'entrée, le deuxième réseau partiel (22) comprenant une deuxième couche d'entrée, le troisième réseau partiel (23) comprenant une couche d'envoi, et le premier réseau partiel (21) et le deuxième réseau partiel (22) étant regroupés dans le troisième réseau partiel (23),
∘ la multitude de clichés radiologiques (24) étant amenée à la première couche d'entrée et la multitude de données vitales (25) étant amenée à la deuxième couche d'entrée,
∘ le premier réseau partiel (21) étant configuré pour générer pour chaque cliché radiologique (24) un descripteur d'image dépendant du temps,
∘ le deuxième réseau partiel (22) étant configuré pour générer à partir des données vitales (25) des descripteurs de données vitales dépendant du temps,
∘ les descripteurs d'image dépendant du temps et les descripteurs de données vitales dépendant du temps étant amenés dans le réseau neuronal artificiel (20), qui comprennent des neurones rétroactifs,
∘ le réseau neuronal artificiel (22) ayant été entraîné au moyen de données de référence pour calculer à l'aide de données de patient une valeur indiquant un SDRA (26) et pour envoyer la valeur indiquant un SDRA (26) par la couche de sortie,
- réception d'une valeur indiquant un SDRA (26) pour les données de patient amenées par le réseau neuronal artificiel (20),
- comparaison de la valeur indiquant un SDRA (26) à une valeur de seuil définie,
- envoi d'une notification si la valeur indiquant un SDRA (26) diffère de la valeur de seuil d'une manière définie.
